# EUROPEAN PATENT APPLICATION

(11) **EP 4 424 818 A1**
(43) Date of publication of application: **04.09.2024**
(21) Application number: 22887139.8
(22) Date of filing: 27.10.2022
(51) Int. Cl.: C12N 5/071, C07K 14/78, C12N 5/0775

(54) **METHOD FOR PRODUCING ORGANISM, AND METHOD FOR PROMOTING DIFFERENTIATION OF HUMAN ADIPOSE-DERIVED STEM CELLS INTO VASCULAR ENDOTHELIAL CELLS**

(30) Priority: 29.10.2021 JP 2021177295
(71) Applicant: Toppan Holdings Inc., Tokyo 110-0016 (JP); OSAKA UNIVERSITY, Suita-shi Osaka 565-0871 (JP); Kyoto Prefectural Public University Corporation, Kyoto-shi, Kyoto 602-8566 (JP)
(72) Inventor: KITANO Shiro, Tokyo 110-0016 (JP); MATSUSAKI Michiya, Suita-shi, Osaka 565-0871 (JP); LOUIS Fiona, Suita-shi, Osaka 565-0871 (JP); SOWA Yoshihiro, Kyoto-shi, Kyoto 602-8566 (JP)
(74) Representative: TBK
(86) International application number: PCT/JP2022/040212
(87) International publication number: WO 2023/074814

(57) **Abstract**

There is provided a method for producing a tissue construct comprising vascular endothelial cells, the method comprising incubating cells comprising at least adipose-derived stem cells from human in the presence of a TGFβ type I receptor inhibitor.

## Description

### Technical Field

The present invention relates to a method for producing a tissue construct, and a method for promoting differentiation of adipose-derived stem cells from human into vascular endothelial cells.

### Background Art

Known as techniques to artificially produce constructs simulating biological tissues are, for example, a method for producing a three-dimensional tissue construct , the method including three dimensionally disposing cells each coated with a coating film containing collagen to form a three-dimensional tissue construct (Patent Literature 1), and the method for producing three-dimensional cellular tissue, the method including: mixing cells with a cationic substance and an extracellular matrix component to give a mixture, collecting cells from the resulting mixture, and forming cell aggregates on a substrate (Patent Literature 2). The present inventors have proposed a method for producing a large-sized three-dimensional tissue construct having a thickness of 1 mm or more with use of a relatively small number of cells by bringing cells and fragmented exogenous collagen into contact (Patent Literature 3).

### Citation List

### Patent Literature

[Patent Literature 1] International Publication No. WO2015/072164
[Patent Literature 2] International Publication No. WO2017/146124
[Patent Literature 3] International Publication No. WO2018/143286

### Summary of Invention

### Technical Problem

According to the production method described above, it is possible to obtain a tissue construct as aggregates of cells artificially produced by cell culture. In particular, a tissue construct containing vascular endothelial cells is expected to be used as a substitute for a laboratory animal, a transplant material, or the like. Here, adipose-derived stem cells derived from other species such as cattle, allogenic cells collected from different individuals even in the case of the human, and the like are often used in artificially producing a tissue construct. However, it is assumed that, for example, in the production of a material for transplantation to a human, a transplant material is produced using autologous cells of the human. Therefore, a method of promoting the differentiation of the adipose-derived stem cells into vascular endothelial cells is desired particularly in a case where a tissue construct is produced using adipose-derived stem cells from human.

The present invention has been made in consideration of the above circumstances, and an object of the present invention is to provide a method of promoting differentiation of adipose-derived stem cells from human into vascular endothelial cells in the production of a tissue construct containing vascular endothelial cells.

### Solution to Problem

As a result of diligent research, the inventors of the present invention found that the differentiation of adipose-derived stem cells into vascular endothelial cells is promoted by incubating adipose-derived stem cells from human in the presence of a TGFβ type I receptor inhibitor, thereby completing the present invention.

That is, the present invention includes, for example, the following inventions.
[1] A method for producing a tissue construct comprising vascular endothelial cells, the method comprising incubating cells comprising at least adipose-derived stem cells from human in the presence of a TGFβ type I receptor inhibitor.
[2] The method of [1], wherein the cells comprising at least adipose-derived stem cells from human comprise no vascular endothelial cells.
[3] The method of [1] or [2], wherein the incubating in the presence of the TGFβ type I receptor inhibitor is incubating in a culture medium comprising the TGFB type I receptor inhibitor, and,
   a content of the TGFβ type I receptor inhibitor in the culture medium is 1 µM or more and 10 µM or less.
[4] The method of any one of [1] to [3], wherein the incubating is performed for 96 hours or more and 288 hours or less.
[5] The method of any one of [1] to [4], comprising incubating the cells together with a fragmented extracellular matrix component in the presence of the TGFβ type I receptor inhibitor.
[6] The method of [5], wherein in the tissue construct, the fragmented extracellular matrix component is disposed in gaps between the cells.
[7] The method of [5] or [6], wherein the fragmented extracellular matrix component is a fragmented collagen component.
[8] The method of any one of [5] to [7], further comprising, before the incubation, a step of bringing the cells into contact with the fragmented extracellular matrix component in an aqueous medium.
[9] A method for promoting differentiation of adipose-derived stem cells into vascular endothelial cells, the method comprising incubating cells comprising at least adipose-derived stem cells from human in the presence of a TGFβ type I receptor inhibitor.
[10] The method of [9], wherein the incubating in the presence of the TGFβ type I receptor inhibitor is incubating in a culture medium comprising the TGFB type I receptor inhibitor, and,
   a content of the TGFβ type I receptor inhibitor in the culture medium is 1 µM or more and 10 µM or less.
[11] The method of [9] or [10], wherein the incubating is performed for 96 hours or more and 288 hours or less.
[12] The method of any one of [9] to [11], comprising incubating the cells comprising at least the adipose-derived stem cells, together with a fragmented extracellular matrix component in the presence of the TGFβ type I receptor inhibitor.
[13] The method of [12], wherein the fragmented extracellular matrix component is a fragmented collagen component.
[14] The method of [12] or [13], further comprising, before the incubation, a step of bringing the cells into contact with the fragmented extracellular matrix component in an aqueous medium.

### Advantageous Effects of Invention

According to the present invention, the differentiation of adipose-derived stem cells from human into vascular endothelial cells is promoted. The promotion of the differentiation into vascular endothelial cells makes it possible to produce a tissue construct containing vascular endothelial cells in a short time.

### Brief Description of Drawings

FIG. 1 shows photographic images that show results obtained by observing tissue constructs prepared in Production Example 1 by CD31 staining and Hoechst staining. (a) shows a tissue construct cultured in EGM-2 that does not contain an ALK5 inhibitor (ALK5i), (b) shows a tissue construct cultured in EGM-2 that contains a 5 µM ALK5 inhibitor, and (c) shows a tissue construct cultured in a DMEM culture medium that contains 10% FBS. White spots indicate nuclei (Hoechst staining).
FIG. 2 shows photographic images that show results obtained by observing tissue constructs prepared in Production Example 2 by CD31 staining.
FIG. 3 shows graphs that show results obtained by analyzing ratios of vascular endothelial cells in tissue constructs prepared in Production Example 3, by flow cytometry analysis, and a table showing the ratios thereof. (a) shows results obtained by using the produced tissue construct, and (b) shows results obtained by using cells as they are, the cells having been isolated after tissue digestion.
FIG. 4 shows photographic images that show results obtained by comparing tissue constructs (cell balls) prepared in Preparation Example 4. The top photographic image shows the produced cell ball(s), the middle photographic image shows cell balls used for CD31 staining analysis, and the bottom photographic image shows results obtained by observing cell balls having a vascular network by CD31 staining.
FIG. 5 shows graphs that show results obtained by analyzing ratios of vascular endothelial cells in tissue constructs prepared in Production Example 5, by flow cytometry analysis, and a table showing the ratios thereof.
FIG. 6 shows photographic images that show results obtained by comparing the formation of the vascular network by vWF staining in tissue constructs (cell balls) prepared in Preparation Example 6.
FIG. 7 shows photographic images that show results obtained by observing tissue constructs (cell balls) prepared in Preparation Example 7.

### Description of Embodiments

Hereinafter, embodiments for executing the present invention will be described in detail. However, the present invention is not limited to the following embodiments.

The present invention provides, as one embodiment, a method for producing a tissue construct comprising vascular endothelial cells, the method comprising incubating cells comprising at least adipose-derived stem cells from human in the presence of a TGFβ type I receptor inhibitor.

In the present specification, the term "tissue construct" means an aggregate of cells artificially produced by cell culture. Among the above, the term "three-dimensional tissue construct" means an aggregate of cells (an aggregated cell population) that is artificially produced by cell culture and in which cells are disposed three-dimensionally. In a case where the three-dimensional tissue construct contains an extracellular matrix component, which is described below, cells are disposed three-dimensionally through the extracellular matrix component. The shape of the tissue construct is not particularly limited, and examples thereof include a sheet shape, a spherical shape, a substantially spherical shape, an ellipsoidal shape, a substantially ellipsoidal shape, a hemispherical shape, a substantially hemispherical shape, a semicircular shape, a substantially semicircular shape, a rectangular shape, and a substantially rectangular shape. Here, the biological tissue includes a sweat gland, a lymphatic vessel, a sebaceous gland, and the like, and the composition thereof is more complex than the tissue construct. Therefore, the tissue construct is easily distinguishable from the biological tissue.

In the present specification, "cell" is not limited; however, it may be a cell derived from a mammalian animal such as a human, a monkey, a dog, a cat, a rabbit, a pig, cattle a mouse, or a rat. The site from which a cell is derived is also not limited. The cell may be a somatic cell derived from bone, muscle, an internal organ, nerve, brain, bone, skin, blood, or the like, or it may be a reproductive cell. Further, the cell may be a stem cell and may be a cultured cell such as a primary cultured cell, a subcultured cell, or a cell lined cell.

In the present specification, the term "stem cell" means a cell that has self-replication ability and pluripotency. The stem cell includes a pluripotent stem cell, which has the ability to differentiate into any cell type, and a tissue stem cell (also referred to as a somatic stem cell), which has the ability to differentiate into a specific cell type. Examples of the pluripotent stem cell include an embryonic stem cell (ES cell), a somatic cell-derived ES cell (ntES cell), and an induced pluripotent stem cell (iPS cell). Examples of the tissue stem cell include a mesenchymal stem cells (for example, an adipose-derived stem cell or a bone marrow-derived stem cell), a hematopoietic stem cell, and a neural stem cell. Examples of the adipose-derived stem cell include a adipose-derived stem cell (ADSC) from human.

The cell includes at least adipose-derived stem cells from human. Regarding the adipose-derived stem cell from human, a stem cell collected from a human biological tissue, for example, a human subcutaneous adipose tissue or a human epicardium-derived adipose tissue may be used. In a case where the tissue construct containing vascular endothelial cells, which is produced by the method according to the present embodiment, is ultimately used as a tissue of a specific site of a human living body, it is preferable to use a tissue derived from a tissue corresponding to the tissue of that site.

In the present embodiment, the cell includes at least a adipose-derived stem cell from human; however, it may further include cells other than the adipose-derived stem cell. Examples of the cells other than the adipose-derived stem cell include a mesenchymal cell such as a mature adipocyte, a vascular endothelial cell, a fibroblast, a chondrocyte, or an osteoblast, a cancer cell such as a colon cancer cell (for example, human colon cancer cell (HT29)) or a liver cancer cell, a myocardial cell, an epithelial cell (for example, a human gingival epithelial cell), a lymphatic vessel endothelial cell, a nerve cell, a dendritic cell, a hepatocyte, an adhesive cell (for example, an immune cell), a smooth muscle cell (for example, an aortic smooth muscle cells (Aorta-SMC)), a pancreatic islet cell, and a keratinocyte (for example, a human epidermal keratinocyte).

The collection of adipose-derived stem cells and/or mature adipocytes from a biological tissue can be carried out according to a conventional method; however, the collection can be carried out, for example, as follows. An adipose tissue fragment is washed and cut and then digested (for example, incubated at 37°C for 1 hour) with collagenase. Thereafter, the digested cells are recovered by filtration or another method, thereby being separated into an oily layer as an upper yellow layer, which contains mature adipocytes, and a pellet as a lower layer, which contains adipose stem cells and blood cells. Mature adipocytes can be collected by recovering only the upper layer, and ADSC can be collected by recovering only the pellet.

In the present specification, "vascular endothelial cell" means a flat-shaped cell that constitutes the surface of the lumen of the blood vessel. Examples of the vascular endothelial cell include a human umbilical vein vascular endothelial cell (HUVEC). Whether adipose stem cells have differentiated into vascular endothelial cells can be confirmed by, for example, immunostaining or flow cytometry analysis using CD31, a von Willebrand factor (vWF), VE-cadherin, and the like, which are markers of vascular endothelial cells.

The content of vascular endothelial cells in the tissue construct may be, for example, 5% or more, 10% or more, 15% or more, 20% or more, 25% or more, or 30% or more, and may be less than 95%, 90%, 80% or 75%, with respect to the number of total cells in the tissue construct. From the viewpoint the effect due to the present invention is more remarkable, the content of the cells including at least adipose-derived stem cells, that is, the vascular endothelial cells in cells before incubation may be less than 10%, 5%, or 1% with respect to the number of total cells before incubation.

The tissue construct may have a vascular network between cells. In a case where a vascular network is formed between cells, it is expected that the tissue construct can be maintained for a long period of time and that the tissue construct is easily engrafted in a case where the tissue construct is transplanted into a mammal or the like.

The phrase "having a vascular network between cells" means having a structure in which branched blood vessels have extended between cells to surround the cells as in the case of the biological tissue. Whether or not the same vascular network as that of the biological tissue has been formed can be determined, for example, based on the diversity of the number of branches and/or the lengths between branches of blood vessels and/or the diameters of blood vessels. For example, in a case where the average value of the number of branches of blood vessels in a tissue construct is 80% or more and 150% or less, 85% or more and 130% less, or 90% or more and 120% or less with respect to the average value of the number of branches of blood vessels in the biological tissue, it may be determined to be similar to the number of branches of blood vessels in the biological tissue. In addition, for example, in a case where the average value of the number of branches of blood vessels in a tissue construct is 2.5 or more and 4.5 or less, or 3.0 or more and 4.2 or less, it may be determined to be similar to the number of branches of blood vessels in the biological tissue. For example, in a case where the average value of the lengths between branches of blood vessels in a tissue construct is 80% or more and 150% or less, 85% or more and 130% less, or 90% or more and 120% or less with respect to the average number of lengths between branches of blood vessels in the biological tissue, it may be determined to be similar to the lengths between branches of blood vessels in the biological tissue. In the biological tissue, both thick and thin blood vessels are observed. Therefore, for example, in a case where both blood vessels having a large diameter (for example, 10 µm or more and less than 25 µm) and blood vessels having a small diameter (for example, more than 0 µm and less than 10 µm) are observed as in the case of the biological tissue, it may be determined that the diversity is the same as the diversity of the diameters of blood vessels in the biological tissue. In addition, for example, in a case where 60% or more, 70% or more, or 80% or more of the diameters of the total blood vessels are distributed in a range of more than 0 µm and less than 25 µm, it may be determined that the diversity is the same as the diversity of the diameters of blood vessels in the biological tissue. It is preferable that a tissue construct containing vascular endothelial cells has a vascular network between the vascular endothelial cells. In that case, in addition to having a vascular network, it is preferable that vascular endothelial cells surrounded by blood vessels are also similar to those in the biological tissue. In a case of comparing the biological tissue and tissue construct described above, the biological tissue and the tissue construct are compared under the same conditions (for example, in terms of a defined volume, a defined area in a case of image analysis, a defined sample, and the like).

The tissue construct may further contain cells other than the vascular endothelial cell. Examples of the cells other than the vascular endothelial cell include a mesenchymal cell such as a mature adipocyte, a fibroblast, a chondrocyte, or an osteoblast, a cancer cell such as a colon cancer cell (for example, human colon cancer cell (HT29)) or a liver cancer cell, a myocardial cell, an epithelial cell (for example, a human gingival epithelial cell), a lymphatic vessel endothelial cell, a nerve cell, a dendritic cell, a hepatocyte, an adhesive cell (for example, an immune cell), a smooth muscle cell (for example, an aortic smooth muscle cells (Aorta-SMC)), a pancreatic islet cell, and a keratinocyte (for example, a human epidermal keratinocyte). However, due to the fact that the effect due to the present invention is more remarkable since an effect of promoting the differentiation of adipose-derived stem cells into vascular endothelial cells is exhibited in the present invention, in the tissue construct after incubation, the number of adipose-derived stem cells is preferably 10% or less and more preferably 5% or less with respect to the number of total cells in the tissue construct, and it is still more preferable that adipose-derived stem cells are not contained.

Fat droplet size can be used as an indicator of adipocyte maturity. The fat droplet is an intracellular organelle that stores lipids such as triglyceride (neutral fat) and cholesterol and has a liquid droplet-like shape since the above lipids are covered with a single membrane of phospholipids. In addition, proteins (perilipin and the like) specific to the adipose tissue are expressed on the surface of the above phospholipids. The sizes of fat droplets in mature adipocytes vary. However, for example, in a case where the average value of the sizes of fat droplets is 20 µm or more, the adipocytes can be regarded as being matured to some extent, that is, the adipocytes can be regarded as mature adipocytes. It is noted that the term "adipocyte" in the present specification means all adipocytes except for adipose-derived stem cells, and includes mature adipocytes and adipocytes that are not included in the adipose-derived stem cells.

The thickness of the tissue construct is preferably 10 µm or more, more preferably 100 µm or more, and still more preferably 1,000 µm or more. Such a tissue construct has a structure more similar to the biological tissue, which makes the tissue construct suitable as a substitute for a laboratory animal and as a transplant material. The maximum thickness of the tissue construct is not limited; however, for example, it may be may be 10 mm or less, may be 3 mm or less, may be 2 mm or less, may be 1.5 mm or less, or may be 1 mm or less.

Here, the term "thickness of the tissue construct" means a distance between the two ends in a direction perpendicular to a principal surface in a case where the tissue construct has a sheet shape or rectangular shape. In a case where the above principal surface is uneven, thickness means a distance at the thinnest portion of the above principal surface. In addition, it means a diameter of a spherical shape or a substantially spherical shape, in a case where the tissue construct has the spherical shape or the substantially spherical shape. Furthermore, it means a short diameter of an ellipsoidal shape or a substantially ellipsoidal shape in a case where the tissue construct has the ellipsoidal shape or the substantially ellipsoidal shape. In a case where the tissue construct has a substantially spherical shape or an ellipsoidal shape and has an uneven surface, thickness means a distance between two points at which a line passing through the centroid of the tissue construct intersects the above surface, where the distance is the shortest distance.

The method according to the present embodiment comprises incubating cells including at least adipose-derived stem cells from human in the presence of a TGFβ type I receptor inhibitor. In a case where adipose-derived stem cells from human are incubated in the presence of a TGFβ type I receptor inhibitor, the differentiation of adipose-derived stem cells from human into vascular endothelial cells is promoted. In the method according to the present embodiment, it is possible to ultimately prepare a tissue construct containing vascular endothelial cells by promoting differentiation in a state where adipose-derived stem cells are contained in the tissue construct, or by promoting differentiation in a state where the tissue construct is formed so that adipose-derived stem cells are contained in the tissue construct. Therefore, it suffices that the production of a tissue construct may include incubating cells including at least adipose-derived stem cells from human in the presence of a TGFβ type I receptor inhibitor, and the production thereof may be carried out by three-dimensional culture or may be carried out by a two-dimensional culture. The tissue construct may be such one that is obtained, for example, by only incubating cells including at least adipose-derived stem cells from human in a culture medium, may be such one that is obtained by suspension culture in which the cells are incubated while being allowed to float in a culture medium in the absence of a scaffold, may be such one that is obtained by allowing the cells to adhere to a scaffold such as a gel or a sheet, or may be such one that is obtained by incubating the cells together with an extracellular matrix in an aqueous medium, as will be described below.

Regarding the incubation of cells including at least adipose-derived stem cells from human in the presence of a TGFβ type I receptor inhibitor, only cells may be incubated in the presence of a TGFβ type I receptor inhibitor, cells and an extracellular matrix component may be incubated in a mixed state in the presence of a TGFβ type I receptor inhibitor, or the incubation may be carried out in the presence of a TGFβ type I receptor inhibitor in a state where a tissue construct containing cells and an extracellular matrix component are formed.

Examples of the TGFβ type I receptor inhibitors include inhibitors of ALK5, ALK2, ALK4, and ALK7, which are TGFβR1 kinases. TGFβ type I receptor inhibitor is not limited as long as it has the ability to inhibit the TGFβ type I receptor. Examples of the ALK5, ALK4, and ALK7 inhibitors include 4-[4-(1,3-benzodioxol-5-yl)-5-(2-pyridinyl)-1H-imidazol-2-yl]benzamid e, and Examples of the ALK5 inhibitor include 2-[3-(6-methyl-2-pyridinyl)-1H-pyrazol-4-yl]-1,5-naphthyridine, 2-(3-(6-methylpyridin-2-yl)-1H-pyrazol-4-yl)-1,5-naphthyridine hydrochloride, and N-(2-fluorophenyl)-4-([1,2,4]triazolo[1,5-a]pyridin-6-yl)-5-(6-methyl-2 -pyridyl)-1H-imidazol-2-methanamine.

The incubating in the presence of the TGFβ type I receptor inhibitor may be, for example, incubating (culturing) cells including at least adipose-derived stem cells from human in a culture medium containing a TGFβ type I receptor inhibitor.

The culture medium is not particularly limited, and a suitable culture medium can be selected according to the kind of cells to be cultured. The culture medium may be a solid culture medium or may be a liquid culture medium; however, it is preferably a liquid culture medium. Examples of the culture medium include an Eagle's MEM medium, a MEM medium, DMEM, a culture medium dedicated to vascular endothelial cells (EGM-2), a Modified Eagle medium (MEM), a Minimum Essential culture medium, RPMI, a KBM medium, and a GlutaMax culture medium. In addition, regarding the liquid culture medium, a gel-like culture medium such as a fibrin gel, a hydrogel, a Matrigel, a collagen gel, or a gelatin gel may be used. Cells may be added to a gel-like liquid culture medium, or a liquid culture medium containing cells may be gelated and used. The culture medium may be a culture medium to which serum has been added or may be a serum-free culture medium. The culture medium may be a mixed culture medium obtained by mixing two kinds of culture media.

It suffices that the amount of the TGFβ type I receptor inhibitor is such an amount that each adipose-derived stem cell sufficiently contacts with the inhibitor, the amount thereof is, for example, may be 2.0 × 10⁻¹¹ mol to 2.0 × 10⁻⁸ mol or 1.0 × 10⁻⁹ mol to 2.0 × 10⁻⁸ mol with respect to 1.0 × 10⁶ cells of adipose-derived stem cells, or may be, for example, 1.0 × 10⁻¹⁰ mol to 1.0 × 10⁻⁷ mol or 5.0 × 10⁻⁹ mol to 1.0 × 10⁻⁷ mol with respect to 5.0 × 10⁶ cells of adipose-derived stem cells. In addition, the TGFβ type I receptor inhibitor may be, for example, 6.0 ng to 6.0 µg or 300 ng to 6.0 µg with respect to 1.0 × 10⁶ cells of adipose-derived stem cells, or may be, for example, 30 ng to 30 µg or 1,500 ng to 30 µg with respect to 5.0 × 10⁶ cells of adipose-derived stem cells.

In a case of incubating cells including at least adipose-derived stem cells from human in a culture medium containing a TGFβ type I receptor inhibitor, the amount of the TGFβ type I receptor inhibitor in the culture medium may be, for example, 0.1 µM or more and 100 µM or less, 0.5 µM or more 50 µM or less, 1 µM or more and 10 µM or less, or 2 µM or more and 8 µM or less, may be 0.5 µM or more, 0.7 µM or more, 1 µM or more, 2 µM or more, 3 µM or more, 4 µM or more, 5 µM or more, or may be 20 µM or less, 15 µM or less, 12 µM or less, 10 µM or less, 8 µM or less, or 7 µM or less.

The cell density in the culture medium before the incubation can be determined according to the shape or thickness of the target tissue construct, the size of the culture vessel, or the like. For example, the cell density in the culture medium may be 1 to 10⁸ cells/mL or may be 10³ to 10⁷ cells/mL.

The incubation is not particularly limited and can be carried out under suitable conditions according to the kind of cells to be cultured. For example, the incubation temperature may be 20°C to 40°C or may be 30°C to 37°C. The pH of the culture medium may be 6 to 8 or may be 7.2 to 7.4. The incubation time may be 24 hours or more and 336 hours or less, may be 72 hours or more and 336 hours or less, or may be 96 hours or more and 288 hours or less. The time of incubation after the addition of the TGFβ type I receptor inhibitor may be 24 hours or more and 336 hours or less, may be 72 hours or more and 336 hours or less, or may be 96 hours or more and 288 hours or less.

The culture vessel (support) that is used for cell culture is not limited and may be, for example, a well insert, a low-adhesion plate, or a plate having a bottom shape such as a U- or V-shape. The above cells may be cultured while being allowed to adhere to a support, the above cells may be cultured without being allowed to adhere to a support, or the above cells may be detached from a support in the middle of the culture and then cultured. In a case of culturing the above cells without allowing them to adhere to a support or in a case of culturing the above cells by detaching them from a support in the middle of the culture, it is preferable to use a plate having a bottom shape such as a U-shape or V-shape, which inhibits the adhesion of cells to a support, or a low-adsorption plate.

In a case where the method according to the present embodiment is incubating (culturing) cells including at least adipose-derived stem cells from human in a culture medium containing a TGFβ type I receptor inhibitor, the culture medium may contain vascular endothelial growth factor (VEGF). In this case, for example, a culture medium containing VEGF and a TGFβ type I receptor inhibitor in advance may be used, a TGFβ type I receptor inhibitor may be added to a culture medium containing VEGF, or VEGF may be added to a culture medium containing a TGFβ type I receptor inhibitor.

In a case where cells including at least adipose-derived stem cells from human are incubated in a culture medium containing a TGFβ type I receptor inhibitor and VEGF, the differentiation of adipose-derived stem cells into vascular endothelial cells is further promoted. In addition, in a case where cells including at least adipose-derived stem cells from human are incubated in a culture medium containing a TGFβ type I receptor inhibitor and VEGF, angiogenesis can also be promoted, and thus it is possible to easily produce a tissue construct having a vascular network.

In a case of incubating cells including at least adipose-derived stem cells from human in a culture medium containing VEGF, the content of VEGF in the culture medium may be, for example, 0.5 ng/mL or more and 100 ng/mL or less, 1 ng/mL or more and 85 ng/mL or less, 6 ng/mL or more and 80 ng/mL or less, 10 ng/mL or more and 70 ng/mL or less, or 20 ng/mL or more and 50 ng/mL or less, may be 0.5 ng/mL or more, 1 ng/mL or more, 5 ng/mL or more, 6 ng/mL or more, 10 ng/mL or more, 15 ng/mL or more, or 20 ng/mL or more, and may be 100 ng/mL or less, 90 ng/mL or less, 80 ng/mL or less, 70 ng/mL or less, 60 ng/mL or less, or 50 ng/mL or less.

The method according to the present embodiment may include incubating cells including at least adipose-derived stem cells from human, together with a fragmented extracellular matrix component in the presence of the TGFβ type I receptor inhibitor. In a case where cells are incubated together with a fragmented extracellular matrix component, it is possible to obtain a three-dimensional tissue construct in which the cells are disposed three-dimensionally through the fragmented extracellular matrix component. The three-dimensional tissue construct is preferably such that the fragmented extracellular matrix component is disposed in the gaps between the cells described above. The cells regarding among the cells may be allogeneic cells or may be heterologous cells. In a case of using a culture medium containing a TGFβ type I receptor inhibitor, the fragmented extracellular matrix component and the TGFβ type I receptor inhibitor may be added to the culture medium at the same time, the fragmented extracellular matrix component may be added to the culture medium before the addition of the TGFβ type I receptor inhibitor, or the fragmented extracellular matrix component may be added to the culture medium after the addition of the TGFβ type I receptor inhibitor.

The fragmented extracellular matrix component can be obtained by fragmenting an extracellular matrix component. In the present specification, the term "extracellular matrix component" is an aggregate of extracellular matrix molecules, which is formed from multiple extracellular matrix molecules. The extracellular matrix means a substance that is present outside of cells in a living organism. Any substance can be used as an extracellular matrix as long as it does not adversely affect cell growth and the formation of cell aggregates. Specific examples thereof include, but are not limited to, collagen, elastin, proteoglycan, fibronectin, hyaluronic acid, laminin, vitronectin, tenascin, entactin, and fibrillin. One kind of extracellular matrix component may be used alone, or two or more kinds thereof may be used in combination. For example, the extracellular matrix component may include a collagen component or may be a collagen component. The extracellular matrix component in the present embodiment is preferably a substance that is present outside of animal cells, that is, an animal extracellular matrix component.

The extracellular matrix molecule may be a modified molecule and a variant of the extracellular matrix molecules described above or may be a polypeptide such as a chemically synthesized peptide, as long as it does not adversely affect cell growth and the formation of cell aggregates. The extracellular matrix molecule may have repeats of a sequence represented by Gly-X-Y, which is characteristic of collagen. Here, Gly represents a glycine residue, and X and Y each independently represent any amino acid residue. A plurality of pieces of Gly-X-Y may be the same or different from each other. In a case where repetitions of the sequence represented by Gly-X-Y are provided, the restriction on the disposition of the molecular chain is reduced and thus, for example, a still more excellent function as a scaffold material in cell culture is obtained. In an extracellular matrix molecule having repeats of the sequence represented by Gly-X-Y, the proportion of the sequence represented by Gly-X-Y may be 80% or more in the total amino acid sequence, and it is preferably 95% or more. In addition, the extracellular matrix molecule may also be a polypeptide having an RGD sequence. The RGD sequence refers to a sequence represented by Arg-Gly-Asp (arginine residue - glycine residue - aspartic acid residue). In a case where the RGD sequence is provided, cell adhesion is much further promoted, and thus, for example, a still more suitable scaffold material in cell culture is obtained. Examples of the extracellular matrix molecule containing the sequence represented by Gly-X-Y and the RGD sequence include collagen, fibronectin, vitronectin, laminin, and cadherin.

Examples of the collagen include a fibrous collagen and a non-fibrous collagen. The fibrous collagen means collagen that is the main component of the collagen fiber, and specific examples thereof include type I collagen, type II collagen, and type III collagen. Examples of the non-fibrous collagen include type IV collagen.

Examples the proteoglycan includes, but are not limited to, chondroitin sulfate proteoglycan, heparan sulfate proteoglycan, keratan sulfate proteoglycan, and dermatan sulfate proteoglycan.

Due to the fact that the effect due to the present invention is more remarkable, the extracellular matrix component may include at least one selected from the group consisting of collagen, laminin, and fibronectin, and it is preferable to include collagen. The collagen is preferably a fibrous collagen and more preferably type I collagen. As the fibrous collagen, a commercially available collagen may be used, and specific examples thereof include a porcine skin-derived type I collagen, manufactured by NH Foods Ltd.

The extracellular matrix component may be an animal-derived extracellular matrix component. Examples of the animal species from which the extracellular matrix component is derived include, but are not limited to, a human, a pig, and cattle. Regarding the extracellular matrix component, a component derived from one species of animal may be used, or components derived from a plurality of animal species may be used in combination.

The term "fragmentation" means reducing a size of an aggregate of an extracellular matrix molecule to a smaller size. Fragmentation may be carried out under conditions in which bonds within an extracellular matrix molecule is cleaved or may be carried out under conditions in which bonds within an extracellular matrix molecule is not cleaved. Unlike enzyme treatment, a molecular structure of an extracellular matrix fragmented by the application of physical force is usually unchanged from the structure before fragmentation (molecular structure is maintained). The fragmented extracellular matrix component may include a defibrated extracellular matrix component (a defibrated extracellular matrix component) which is a component obtained by defibrating the extracellular matrix component described above by the application of physical force. Defibration is one aspect of fragmentation and is carried out, for example, under conditions in which bonds within an extracellular matrix molecule are not cleaved.

A method of fragmenting an extracellular matrix component is not limited. Regarding a method of defibrating an extracellular matrix component, the extracellular matrix component may be defibrated by the application of physical force with, for example, an ultrasonic homogenizer, a stirring type homogenizer, or a high-pressure homogenizer. In a case where a stirring type homogenizer is used, the extracellular matrix component may be homogenized as it is or may be homogenized in an aqueous medium such as saline. In addition, it is also possible to obtain a millimeter-sized or nanometer-sized defibrated extracellular matrix component by adjusting the homogenization time, the number of times of homogenization, or the like. The defibrated extracellular matrix component can also be obtained by carrying out defibration by repeated freezing and thawing.

The fragmented extracellular matrix component may include, at least as a part thereof, an extracellular matrix component obtained by defibration. In addition, the fragmented extracellular matrix component may consist of only the extracellular matrix component obtained by defibration. That is, the fragmented extracellular matrix component may be an extracellular matrix component obtained by defibration. The extracellular matrix component obtained by defibration preferably includes a collagen component (defibrated collagen component) obtained by defibration. The defibrated collagen component preferably maintains the triple-helix structure derived from collagen. The defibrated collagen component may be a component that partially maintains the triple-helix structure derived from collagen.

Examples of the shape of the fragmented extracellular matrix component include a fibrous shape. The fibrous shape means a shape that is composed of thread-shaped collagen components or a shape that is composed of thread-shaped extracellular matrix components that have undergone intermolecular crosslinking. At least a part of the fragmented extracellular matrix component may have a fibrous shape. The fibrous extracellular matrix component includes a thin thread-shaped material (fine fibers) formed by aggregation of a plurality of thread-shaped extracellular matrix molecules, a thread-shaped material formed by further aggregation of fine fibers, materials obtained by defibrating these thread-shaped materials, and the like. The RGD sequence is conserved in the fibrous extracellular matrix component without being disrupted.

The average length of the fragmented extracellular matrix component may be 100 nm or more and 400 µm or less or may be 100 nm or more and 200 µm or less. In one embodiment, the average length of the fragmented extracellular matrix component may be 5 µm or more and 400 µm or less, may be 10 µm or more and 400 µm or less, may be 22 µm or more and 400 µm or less, or may be 100 µm or more and 400 µm or less. In another embodiment, the average length of the fragmented extracellular matrix component may be 100 µm or less, may be 50 µm or less, may be 30 µm or less, may be 15 µm or less, may be 10 µm or less, or may be 1 µm or less, and it may be 100 nm or more, from the viewpoint of still more excellent redispersibility. It is preferable that the average length of the majority of the fragmented extracellular matrix components among the total fragmented extracellular matrix components is within the above-described range of numerical values. Specifically, it is preferable that the average length of 95% of the fragmented extracellular matrix components among the total fragmented extracellular matrix components is within the above-described range of numerical values. The fragmented extracellular matrix component is preferably a fragmented collagen component of which the average length is within the above-described range, and it is more preferably a defibrated collagen component of which the average length is within the above-described range.

The average diameter of the fragmented extracellular matrix component may be 10 nm or more and 30 µm or less, may be 30 nm or more and 30 µm or less, may be 50 nm or more and 30 µm or less, may be 100 nm or more and 30 µm or less, may be 1 µm or more and 30 µm or less, may be 2 µm or more and 30 µm or less, may be 3 µm or more and 30 µm or less, may be 4 µm or more and 30 µm or less, and may be 5 µm or more and 30 µm or less. The fragmented extracellular matrix component is preferably a fragmented collagen component of which the average diameter is within the above-described range, and it is more preferably a defibrated collagen component of which the average diameter is within the above-described range.

The average length and diameter of the fragmented extracellular matrix component can be determined by measuring an individual fragmented extracellular matrix component with an optical microscope and carrying out image analysis. In the present specification, "average length" means the average value of lengths of a measured specimen in the longitudinal direction, and "average diameter" means the average value of lengths of a measured specimen in a direction orthogonal to the longitudinal direction.

For example, the fragmented extracellular matrix component may include a fragmented collagen component, and it may consist of a fragmented collagen component. The term "fragmented collagen component" means a component obtained by fragmenting a collagen component such as a fibrous collagen component, where the fragmented collagen component is a component that maintains the triple-helix structure. The average length of the fragmented collagen component is preferably 100 nm to 200 µm, more preferably 22 µm to 200 µm, and still more preferably 100 µm to 200 µm. The average diameter of the fragmented collagen component is preferably 50 nm to 30 µm, more preferably 4 µm to 30 µm, and still more preferably 20 µm to 30 µm.

At least a part of the fragmented extracellular matrix component may be crosslinked intermolecularly or intramolecularly. In the extracellular matrix component, the extracellular matrix molecules that constitute the extracellular matrix component may be crosslinked intramolecularly or intermolecularly.

Examples of the method of carrying out crosslinking include methods by chemical crosslinking and the like with physical crosslinking by application of heat, ultraviolet rays, radiation, and the like, a crosslinking agent, an enzymatic reaction, and the like; however, the method is not limited. Physical crosslinking is preferable from the viewpoint that cell growth is not disturbed. Crosslinking (physical crosslinking and chemical crosslinking) may be covalent bond-mediated crosslinking.

In a case where the extracellular matrix component includes a collagen component, crosslinks may be formed between collagen molecules (triple-helix structures) or may be formed between collagen fine fibers formed from collagen molecules. Crosslinking may be crosslinking by heat (thermal crosslinking). Thermal crosslinking can be carried out, for example, by carrying out a heating treatment under reduced pressure using a vacuum pump. In a case of carrying out thermal crosslinking of a collagen component, an amino group of the collagen molecule may form a peptide bond (-NH-CO-) with a carboxy group of the same or another collagen molecule, thereby the extracellular matrix component being crosslinked.

The extracellular matrix component can also be crosslinked by using a crosslinking agent. The crosslinking agent may be, for example, a crosslinking agent that is capable of crosslinking a carboxyl group to an amino group, or a crosslinking agent that is capable of crosslinking amino groups to each other. The crosslinking agent is, for example, preferably an aldehyde-based, carbodiimide-based, epoxide-based, or imidazole-based crosslinking agent from the viewpoints of economy, safety, and operability. Specific examples thereof include glutaraldehyde, 1-ethyl-3-(3-dimethylaminopropyl)carbodiimide·hydrochloride, and a water-soluble carbodiimide such as 1-cyclohexyl-3-(2-morpholinyl-4-ethyl)carbodiimide sulfonate.

The quantification of the degree of crosslinking can be appropriately selected according to the kind of extracellular matrix component, the means for crosslinking, and the like. The degree of crosslinking may be 1% or more, 2% or more, 4% or more, 8% or more, or 12% or more, and may be 30% or less, 20% or less, or 15% or less. In a case where the degree of crosslinking is in the above-described range, it is possible to properly disperse the extracellular matrix molecule, and redispersibility after dry storage is favorable.

In a case where an amino group in the extracellular matrix component is used for crosslinking, the degree of crosslinking can be quantified based on a TNBS (trinitrobenzenesulfonic acid) method. The degree of crosslinking according to the TNBS method may be within the range described above. The degree of crosslinking according to the TNBS method is the proportion of amino groups used for crosslinking among the amino groups contained in the extracellular matrix. In a case where the extracellular matrix component includes a collagen component, the degree of crosslinking, which is measured according to the TNBS method is preferably within the above-described range.

The degree of crosslinking may be calculated by quantifying carboxyl groups. For example, in a case of a water-insoluble extracellular matrix component, the quantification may be carried out according to a TBO (toluidine blue O) method. The degree of crosslinking according to the TBO method may be within the range described above.

The content of the extracellular matrix component in the three-dimensional tissue construct may be 0.01% to 90% by mass based on the above three-dimensional tissue construct (in terms of dry weight), and it is preferably 10% to 90% by mass, preferably 10% to 80% by mass, preferably 10% to 70% by mass, preferably 10% to 60% by mass, preferably 1% to 50% by mass, preferably 10% to 50% by mass, more preferably 10% to 30% by mass, and still more preferably 20% to 30% by mass.

Here, the phrase "the extracellular matrix component in the three-dimensional tissue construct" means an extracellular matrix component that constitutes a three-dimensional tissue construct, where "the extracellular matrix component in the three-dimensional tissue construct" may be derived from an endogenous extracellular matrix component or may be derived from an exogenous extracellular matrix component.

The term "endogenous extracellular matrix component" means an extracellular matrix component that is produced by extracellular matrix-producing cells. Examples of the extracellular matrix-producing cell include the mesenchymal cells such as the fibroblast, the chondrocyte, and the osteoblast, which are described above. The endogenous extracellular matrix component may have a fibrous shape or a non-fibrous shape.

The term "exogenous extracellular matrix component" means an extracellular matrix component that is supplied from the outside. The exogenous extracellular matrix component may be the same or different from the endogenous extracellular matrix component in terms of the animal species from which it is derived. Examples of the animal species as the origin include a human, a pig, and cattle In addition, the exogenous extracellular matrix component may also be an artificial extracellular matrix component.

In a case where the extracellular matrix component is a collagen component, the exogenous extracellular matrix component is also referred to as an "exogenous collagen component". The "exogenous extracellular matrix component" means a collagen component supplied from the outside is an aggregate of collagen molecules, which is formed from multiple collagen molecules, and specific examples thereof include a fibrous collagen and a non-fibrous collagen. The exogenous collagen component is preferably a fibrous collagen. The fibrous collagen means a collagen component that is the main component of the collagen fiber, and examples thereof include type I collagen, type II collagen, and type III collagen. As the fibrous collagen, a commercially available collagen may be used, and specific examples thereof include a porcine skin-derived type I collagen, manufactured by NH Foods Ltd. Examples of the exogenous non-fibrous collagen include type IV collagen.

In the exogenous extracellular matrix component, the animal species from which it is derived may be different from the species in the case of the cell. In addition, in a case where the cells include extracellular matrix-producing cells, in the exogenous extracellular matrix component, the animal species from which it is derived may be different from the species in the case of the extracellular matrix-producing cell. In other words, the exogenous extracellular matrix component may be a heterogeneous extracellular matrix component.

That is, in a case where the three-dimensional tissue construct contains an endogenous extracellular matrix component and a fragmented extracellular matrix component, the content of the extracellular matrix component that constitutes the three-dimensional tissue construct means the total amount of the endogenous extracellular matrix component and the fragmented extracellular matrix component. The above-described content of the extracellular matrix content can be calculated from the volume of the obtained three-dimensional tissue construct and the mass of the decellularized three-dimensional tissue construct.

For example, in a case where the extracellular matrix component contained in the three-dimensional tissue construct is a collagen component, examples of the method of quantifying the amount of collagen component in the three-dimensional tissue construct include such a method of quantifying hydroxyproline as described below. Hydrochloric acid (HCl) is mixed in a lysis solution in which a three-dimensional tissue construct has been lyzed, the resultant mixture is incubated at high temperature for a predetermined time and then returned to room temperature, and the supernatant obtained by centrifugation is diluted to a predetermined concentration to prepare a sample. A hydroxyproline standard solution is treated in the same manner as in the case of the sample and then serially diluted to prepare a standard. Each of the sample and the standard is subjected to a predetermined treatment with a hydroxyproline assay buffer and a detection reagent to measure an absorbance at 570 nm. The absorbance of the sample is compared with the absorbance of the standard to calculate the amount of collagen component. It is noted that a lysis solution, in which the three-dimensional tissue construct has been lyzed by being directly suspended in hydrochloric acid having a high concentration, may be centrifuged to recover the supernatant, which is subsequently used for the quantification of the collagen component. In addition, the three-dimensional tissue construct to be lyzed may be in the same state as it has been revered from the culture solution, or it may be subjected to a drying treatment after recovery and lyzed in a state where the liquid component has been removed. However, in a case where a three-dimensional tissue construct in the same state as it has been recovered from the culture solution is lyzed to carry out the quantification of the collagen component, the variation in the measured value of the weight of the three-dimensional tissue construct is expected due to the influence of the culture medium components absorbed by the three-dimensional tissue construct and the remaining of the culture medium due to problems in experimental techniques. Therefore, the quantification is preferably based on the weight after drying from the viewpoint of stably measuring the weight of the three-dimensional tissue construct and the amount of collagen component per unit weight.

More specific examples of the method of quantifying the amount of collagen component include such a method described below.

### (Preparation of sample)

An entire amount of a three-dimensional tissue construct which has been subjected lyophilization treatment is mixed with 6 mol/L HCl, incubated in a heating block at 95°C for 20 hours or more and then returned to room temperature. After centrifugation at 13,000g for 10 minutes, the supernatant of the sample solution is recovered. An appropriate dilution is carried out with 6 mol/L HCl so that the results in the measurement described later are within the range of the calibration curve, and then 200 µL of the diluted solution is further diluted with 100 µL of ultrapure water to prepare a sample. 35 µL is sued as a sample.

### (Preparation of standard)

125 µL of a standard solution (1,200 µg/mL in acetic acid) and 125 µL of 12 mol/l HCl are added to a screw cap tube and mixed, incubated in a heating block at 95°C for 20 hours, and then returned to room temperature. After centrifugation at 13,000g for 10 minutes, the supernatant is diluted with ultrapure water to prepare a 300 µg/mL S1, and the S1 is serially diluted to produce an S2 (200 µg/mL), an S3 (100 µg/mL), an S4 (50 µg/mL), an S5 (25 µg/mL), an S6 (12.5 µg/mL), and an S7 (6.25 µg /mL). An S8 (0 µg/mL) of only 90 µL of 4 mol/l HCl only is also prepared.

### (Assay)

35 µL of each of the standard and the sample is added to a plate (attached to QuickZyme Total Collagen Assay kit, QuickZyme Biosciences). 75 µL of an assay buffer (attached to the kit above) to each well. The plate is closed with a seal, and incubation is carried out at room temperature with shaking for 20 minutes. The seal is removed, and 75 µL of a detection reagent (reagent A:B = 30 µL:45 µL, attached to the kit above) is added to each well. The plate is closed with a seal, and the solution is mixed by shaking and incubated at 60°C for 60 minutes. Cooling is sufficiently carried out on ice, and the seal is removed to measure an absorbance at 570 nm. The absorbance of the sample is compared with the absorbance of the standard to calculate the amount of collagen component.

The collagen component in the three-dimensional tissue construct may be specified by the area ratio or volume ratio thereof. The phrase "specified by the area ratio or volume ratio" means that, for example, the collagen component in the three-dimensional tissue construct is made to be in a state of being distinguishable from other tissue constituents by a known staining method (for example, immunostaining using an anti-collagen antibody or Masson's trichrome staining), and then the ratio of the region in which the collagen component is present, in the entire three-dimensional tissue construct, is calculated by using observation by naked eyes, various microscopes, image analysis software, or the like. In a case of being specified by an area ratio, what cross section or surface in the three-dimensional tissue construct specifies the area ratio is not limited. However, the specification may be made with a cross-sectional view that is taken to pass through the central part of three-dimensional tissue construct, for example, in a case where the three-dimensional tissue construct has a spherical shape or the like.

For example, in a case where the collagen component in the three-dimensional tissue construct is specified by the area ratio, the proportion of the area thereof is 0.01% to 99% based on the total area of the three-dimensional tissue construct, and it is preferably 1% to 99%, preferably 5% to 90%, preferably 7% to 90%, preferably 20% to 90%, or more preferably 50% to 90%. "The collagen component in three-dimensional tissue construct" is described above. The proportion of the area of the collagen component that constitutes the three-dimensional tissue construct means the proportion of the combined area of the endogenous collagen component and the exogenous collagen component. The proportion of the area of the collagen component can be obtained, for example, by staining the obtained three-dimensional tissue construct with Masson trichrome and carrying out a calculation to determine a proportion of an area of the blue-stained collagen component with respect to a total area of a cross section that is taken to pass through the central part of three-dimensional tissue construct.

In the three-dimensional tissue construct, the residual rate after trypsin treatment at a trypsin concentration of 0.25%, a temperature of 37°C, a pH of 7.4, and a reaction time of 15 minutes is preferably 70% or more, more preferably 80% or more, and still more preferably 90% or more. In such a three-dimensional tissue construct, the enzymatic degradation is less likely to occur during culture or after culture, and thus the three-dimensional tissue construct is stable. The above residual rate can be calculated, for example, from the masses of the three-dimensional tissue construct before and after trypsin treatment.

In the three-dimensional tissue construct, the residual rate after collagenase treatment at a collagenase concentration of 0.25%, a temperature of 37°C, a pH of 7.4, and a reaction time of 15 minutes may be 70% or more, and it is more preferably 80% or more and still more preferably 90% or more. In such a three-dimensional tissue construct, the enzymatic degradation is less likely to occur during culture or after culture, and thus the three-dimensional tissue construct is stable.

The incubating cells including at least adipose-derived stem cells from human, together with a fragmented extracellular matrix component in the presence of the TGFβ type I receptor inhibitor may be, for example, incubating (culturing) cells in a culture medium containing a TGFβ type I receptor inhibitor and a fragmented extracellular matrix component. In this case, for example, a culture medium containing in advance a fragmented extracellular matrix component and a TGFβ type I receptor inhibitor may be used, a TGFβ type I receptor inhibitor may be added to a culture medium containing a fragmented extracellular matrix component, or a fragmented extracellular matrix component may be added to a culture medium containing a TGFβ type I receptor inhibitor.

A step of bringing the cells into contact with the fragmented extracellular matrix component in an aqueous medium may be further provided before the incubation. In this case, the TGFβ type I receptor inhibitor may be contained in the aqueous medium, may be added thereto before incubation, or may be added thereto during incubation. The aqueous medium may be the same as or may be different from the aqueous medium at the time of the incubation. In a case where the cells are brought into contact with the fragmented extracellular matrix component in an aqueous medium, it is possible to produce a three-dimensional tissue construct in which the fragmented extracellular matrix component is disposed in the gaps between the above cells, and the cells and the fragmented extracellular matrix component are uniformly distributed three dimensionally throughout the three-dimensional tissue construct. For example, after being brought into contact with the fragmented extracellular matrix component in an aqueous medium, the cells may be further incubated in the presence of a TGFβ type I receptor inhibitor. The time for bringing the cells into contact with the fragmented extracellular matrix component in an aqueous medium and then incubating the cells may be, for example, 12 hours to 72 hours.

The term "aqueous medium" means a liquid in which water is an essential constitutional component. The aqueous medium is not particularly limited as long as the fragmented extracellular matrix component can be present stably. Examples the aqueous medium include, but are not limited to, saline such as phosphate buffered saline (PBS) and a liquid culture medium such as a Dulbecco's Modified Eagle medium (DMEM) or a culture medium dedicated to vascular endothelial cells (EGM-2).

The pH of the aqueous medium is preferably in a range in which cell growth and the formation of cell aggregates are not adversely affected. From the viewpoint of reducing the load on cells in a case of being charged into the cells, the pH of the aqueous medium may be, for example, 7.0 or more or may be 8.0 or less, and specifically, the pH of the aqueous medium may be 7.0, 7.1, 7.2, 7.3, 7.4, 7.5, 7.6, 7.7, 7.8, 7.9, or 8.0. The aqueous medium preferably has a buffering capacity in the above-described pH range and is more preferably a liquid culture medium. The liquid culture medium is not particularly limited, and a suitable culture medium can be selected according to the kind of cells to be cultured. Examples of the culture medium include an Eagle's MEM medium, a MEM medium, DMEM, EGM-2, a Modified Eagle culture medium (MEM), a Minimum Essential culture medium, RPMI, and a GlutaMax culture medium. The culture medium may be a culture medium to which serum has been added or may be a serum-free culture medium. Further, the liquid culture medium may be a mixed culture medium obtained by mixing two or more kinds of culture media.

In a case of being dispersed in an aqueous medium, the fragmented extracellular matrix component easily contacts with cells in the aqueous medium and can promote the formation of three-dimensional tissue construct. Examples of the method for the contacting step include, but are not limited to, methods such as a method of mixing an aqueous medium containing a fragmented extracellular matrix component with an aqueous medium containing cells, a method of adding cells to an aqueous medium containing a fragmented extracellular matrix component, a method of adding an aqueous medium containing an extracellular matrix component to a culture solution containing cells, a method of adding cells to an aqueous medium containing an extracellular matrix component, and a method of adding an extracellular matrix component and cells respectively to aqueous media prepared in advance.

The concentration of the fragmented extracellular matrix component in the contacting step can be determined according to the shape or thickness of the target three-dimensional tissue construct, the size of the culture vessel, or the like. For example, in the contacting step, the concentration of the fragmented extracellular matrix component in the aqueous medium may be 0.1% to 90% by mass or may be 1% to 30% by mass.

The amount of the fragmented extracellular matrix component in the contacting step may be, for example, 0.1 to 100 mg, 0.5 to 50 mg, 0.8 to 25 mg, 1.0 to 10 mg, 1.0 to 5.0 mg, 1.0 to 2.0 mg, or 1.0 to 1.8, may be 0.7 mg or more, 1.1 mg or more, 1.2 mg or more, 1.3 mg or more, or 1.4 mg or more, and may be 7.0 mg or less, 3.0 mg or less, 2.3 mg or less, 1.8 mg or less, 1.7 mg or less, 1.6 mg or less, or 1.5 mg or less, with respect to the cells of 1.0 × 10⁶ cells.

In the contacting step, the mass ratio of the fragmented extracellular matrix component to the cells (fragmented extracellular matrix component/cell) is preferably 1/1 to 1,000/1, more preferably 9/1 to 900/1, and still more preferably 10/1 to 500/1.

In addition, the amount of the fragmented extracellular matrix component in the contacting step may be 0.1% to 10% by weight, 0.5% to 8% by weight, or 1% to 5% by weight, with respect to the weight of the entire culture medium.

One embodiment of the method for producing a three-dimensional tissue construct may include mixing fibrinogen and thrombin simultaneously or separately in the contacting step, or after the contacting step and before the incubation. In a case where fibrinogen and thrombin are mixed, they react to form fibrin. For example, the content of fibrinogen may be 1 to 10 mg/mL, may be 2 to 8 mg/mL, or may be 5 to 6 mg/mL, with respect to the culture medium.

A step of precipitating together the fragmented extracellular matrix component and cells in the aqueous medium may be further provided after the contacting step and before the incubation. In a case where such a step is carried out, the distribution of the fragmented extracellular matrix component and the cells in the three-dimensional tissue construct becomes more uniform. The specific method therefor is not particularly limited; however, examples thereof include a method of subjecting a culture solution containing a fragmented extracellular matrix component and cells, to a centrifugal operation.

The cell density in the culture medium before the incubation described above may be the same as the cell density in the aqueous medium in the contacting step.

The present invention also provides, as one embodiment, a method for promoting differentiation of adipose-derived stem cells from human into vascular endothelial cells, which includes incubating cells including at least adipose-derived stem cells from human in the presence of a TGFβ type I receptor inhibitor. As described above, it is possible to promote the differentiation of adipose-derived stem cells from human into vascular endothelial cells by incubating adipose-derived stem cells in the presence of a TGFβ type I receptor inhibitor. The method according to the present embodiment includes promoting differentiation in a state where adipose-derived stem cells from human are contained in the tissue construct, or promoting differentiation in a state where the tissue construct is formed so that adipose-derived stem cells from human are contained in the tissue construct, which makes it possible to ultimately prepare a tissue construct containing vascular endothelial cells.

In the method according to the present embodiment, it suffices that at least a part of adipose-derived stem cells from human is promoted to differentiate by coming in contact with a TGFβ type I receptor inhibitor. Therefore, the method according to the present embodiment can be applied not only in three-dimensional culture in the production of a three-dimensional tissue construct described above but also in another three-dimensional culture and two-dimensional culture.

In addition, the method according to the present embodiment may also be a method for promoting the differentiation of human adipose-derived stem cell into vascular endothelial cells in the method for producing a three-dimensional tissue construct described above.

As described above, in a case where cells including at least adipose-derived stem cells from human are incubated in a culture medium containing a TGFβ type I receptor inhibitor and VEGF, it is possible to promote differentiation of adipose stem cells into vascular endothelial cells and angiogenesis. That is, the method according to the present embodiment can also be used as a method for promoting angiogenesis, which includes incubating (culturing) cells including at least adipose-derived stem cells from human in a culture medium containing a TGFβ type I receptor inhibitor and VEGF. The promotion of angiogenesis may be a promotion of vascular network formation.

The above-described specific aspects and the like can be applied, without limitation, to the specific aspects in the method according to the present embodiment, regarding the tissue construct (including the three-dimensional tissue construct), the cells, the incubation, the fragmented extracellular matrix component, each of the steps, and the like. For example, the method according to the present embodiment may also include a step of bringing the cells into contact with the fragmented extracellular matrix component in an aqueous medium.

### [Examples]

Hereinafter, the present invention will be more specifically described based on Examples. However, the present invention is not limited to Examples below.

Cells, reagents, and preparation methods, which are used in the preparation of a tissue construct, are as follows.

### (Cell and collagen)

. Primary human mature adipocyte and human adipose stem cell (ADSC): Collected from a human adipose tissue (derived from the thigh) (provided by University Hospital, Kyoto Prefectural University of Medicine) according to the method described below
. Human umbilical vein-derived vascular endothelial cell (HUVEC) (manufactured by Lonza, #C-2517A)
. Defibrated collagen component (sCMF) (prepared according to the method described below)

### (Reagent, culture medium, and various solutions)

ALK5 inhibitor: 2-[3-(6-methyl-2-pyridinyl)-1H-pyrazol-4-yl]-1,5-naphthyridine, Cayman Chemical Company, No. 14794
· Bovine pancreas-derived insulin (Sigma-Aldrich Co., LLC, #I1882)
· Lyophilized bovine blood plasma-derived thrombin powder

### (Sigma-Aldrich Co., LLC, #T4648)

· Bovine blood plasma-derived fibrinogen I-S type (Sigma-Aldrich Co., LLC, #F8630)
· Collagenase I derived from Clostridium histolyticum (Sigma-Aldrich Co., LLC, #C0130)
· DMEM (culture medium) (high glucose, Nacalai Tesque, Inc.)
· EGM-2 (culture medium): A culture medium obtained by mixing EGM-2 supplement growth factors (factors (Lonza, #C-2517A)) in 500 mL EBM-2 and storing the resultant mixture at 4°C
· KBM (culture medium) (Kohjin Bio Co., Ltd.)
· 10 mg/mL insulin stock solution: A solution obtained by dissolving 100 mg of the above bovine pancreas-derived insulin in 10 mL of 1% glacial acetic acid solution (pH ≤ 2) diluted with water, followed by aliquoting in Eppendorf tubes and storing at -20°C
· 2 mg/mL collagenase solution: 2.5 g of BSA is mixed with 50 mL of DMEM (0% FBS, 1% antibiotics). A solution, which is obtained by mixing 26 mg of collagenase type I in 13 mL of DMEM (0% FBS, 5% BSA, 1% antibiotics) and filtering the resultant mixture through a filter having a pore diameter of 0.2 µm, is used to digest the entire adipose tissue in a 6-well plate.
· 50 mg/mL fibrinogen stock solution: 50 mg of fibrinogen is weighed into an Eppendorf tube, and 1 mL of DMEM (0% FBS, 1% antibiotics) is immediately added thereto. A solution, which is obtained by carrying out mixing by manually shaking the tube, subsequently placing the tube in a 37°C water bath for 3 to 5 minutes, and carrying out filtering with a filter having a pore diameter of 0.2 µm, followed by aliquoting in Eppendorf tubes, is used.
· 202 U/mL thrombin stock solution: 202 U of thrombin is weighed into an Eppendorf tube, and 1 mL of DMEM (0% FBS, 1% antibiotics) is immediately added thereto and placed in a 37°C water bath for 3 to 5 minutes to dissolve the thrombin. Then, filtration is carried out with a filter having a pore diameter of 0.2 µm, followed by aliquoting in Eppendorf tubes, whereby a solution to be used is obtained.

### (Collagen component obtained by defibration)

100 mg of porcine skin-derived collagen type I sponged fragments (manufactured by NH Foods Ltd.) was heated at 200°C for 24 hours to obtain a collagen component, at least a part of which is crosslinked (a crosslinked collagen component). It is noted that no significant change in the appearance of the collagen was observed before and after heating at 200°C. 50 mg of the crosslinked collagen component was placed in a 15-mL tube, 5 mL of ultrapure water was added thereto, and homogenization was carried out for 6 minutes with a homogenizer (AS ONE Corporation, VH-10) to defibrate the crosslinked collagen component.

Centrifugation was carried out at 10,000 rpm for 10 minutes under the condition of 21°C. The supernatant was aspirated and the collagen pellet was mixed with 5 mL of fresh ultrapure water to prepare a collagen solution. The following operations were repeated 100 times: A tube containing the collagen solution was subjected to sonication treatment at 100 V for 20 seconds using a sonicator (Sonics & Materials, Inc., VC50) while the tube containing the collagen solution was maintained on ice, and the tube containing the collagen solution was taken out from the sonicator and then cooled on ice for 10 seconds. After 100 times of the sonication treatments, the collagen solution was filtered through a filter having a pore diameter of 40 µm to obtain a dispersion liquid containing a defibrated collagen component (CMF). The dispersion liquid was lyophilized according to the conventional method to obtain a lyophilized collagen component (CMF) as a dried product. The average length (length) of the CMF was 14.8 ± 8.2 µm (N = 20).

### (Method for collecting mature adipocyte and ADSC)

A human adipose tissue fragment was washed with PBS containing 5% antibiotics. Four to six grams of the tissue was divided into six wells of a six-well plate. Using scissors and tweezers, the tissue was chopped into small pieces to have a size of approximately 1 to 3 mm in 2 mL of a 2 mg/mL collagenase solution. After incubation at 37°C and 230 rpm for 1 hour, mixing was carried out with a 10-mL pipette for 30 minutes. The lysate was filtered through an iron mesh filter having a pore diameter of 500 µm, 2 mL of DMEM per well was added thereto to recover all the digested cells, and then centrifugation was carried out at 200 g for 3 minutes at room temperature (15°C to 25°C). The oily layer, as the upper yellow layer, contains mature adipocytes, and the pellet contains adipose stem cells and blood cells. Using a long needle and a 10 mL syringe, the medium between the upper and lower layers was aspirated and discarded, and the mature adipocytes contained in the upper layer as well as the adipose stem cells and the blood cells contained in the lower layer were washed twice with 25 mL PBS (5% BSA, 1% antibiotics). In a case of carrying out washing, the same centrifugation as described above was carried out to carry out separation into three layers of the upper layer, the lower layer, and a medium between the upper layer and the lower layer, and then the medium between the upper and lower layers was aspirated and discarded. After the two times of washing, washing was carried out with 25 mL DMEM.

Only the upper layer containing mature adipocytes was collected and dispensed into Eppendorf tubes. Nuclei were stained by Hoechst staining (1,000-fold diluted Hoechst, staining for 15 min), and the number of cells was counted on a Turker Burk hemocytometer using a fluorescence microscope.

The pellet containing the ADSC was suspended in 10 mL of DMEM, and the suspended cells were seeded in a 10-cm dish and subcultured. The ADSC was separated from the dish using trypsin/EDTA and suspended in 1 mL of DMEM, and the number of cells was counted.

### <Production of tissue construct>

### Production Example 1

Isolated ADSC was suspended in a culture medium of 2 mL of EGM-2 containing 0 µM or 5 µM of an ALK5 inhibitor (2-[3-(6-methyl-2-pyridinyl)-1H-pyrazol-4-yl]-1,5-naphthyridine, Cayman Chemical Company, No. 14794) or 2 mL of DMEM containing 10% FBS and seeded in a 24-well plate (IWAKI CO., LTD.) at a concentration of 10,000 cells/well and incubated at 37°C.

The culture medium was changed every 2 days, and culture was carried out for 7 days after seeding to obtain a tissue construct.

Fluorescence imaging of the tissue construct was carried out according to the following procedure. A transwell containing the obtained tissue construct was transferred to a 24-well plate. After washing with 2 mL of PBS, the tissue was fixed overnight under the condition of 4°C using 2 mL of 4% PFA. Washing was carried out three times using 2 mL of PBS. Cells were subjected to permeabilization treatment for 7 minutes under room temperature conditions by using 0.05% Triton/PBS (500 µL in the transwell and 500 µL outside the transwell), and washing was carried out three times using 2 mL of PBS.

The tissue was subjected to blocking treatment for 1 hour under room temperature conditions by using a 1% BSA/PBS solution (500 µL in the transwell and 500 µL outside the transwell). The BSA solution was aspirated, and 100 µL of a primary antibody solution (an anti-CD31 antibody diluted 100-fold in a 1% BSA/PBS solution) was added (50 µL in the transwell and 50 µL outside the transwell). A wet tissue paper was placed under the plate, and the plate was covered with an aluminum foil and incubated overnight under the condition of 4°C. After washing was carried out three times using 2 mL of PBS, 100 µL of a secondary antibody solution (an Alexa Fluor 647-labeled anti-mouse IgG antibody diluted 200-fold in a 1% BSA/PBS solution and 1,000-fold diluted Hoechst) was added thereto (50 µL in the transwell and 50 µL outside the transwell). A wet tissue paper was placed under the plate, the plate was covered with an aluminum foil and incubated for 2 hours under room temperature conditions, and then washing was carried out four times using 2 mL of PBS.

The membrane of the transwell was cut, the gel was placed directly on the bottom of a glass bottom dish containing a small amount of PBS, and the stained tissue construct was observed using a confocal laser scanning microscope (FV3000, manufactured by Olympus Corporation) with laser excitation light at 640 nm (Alexa Fluor 647).

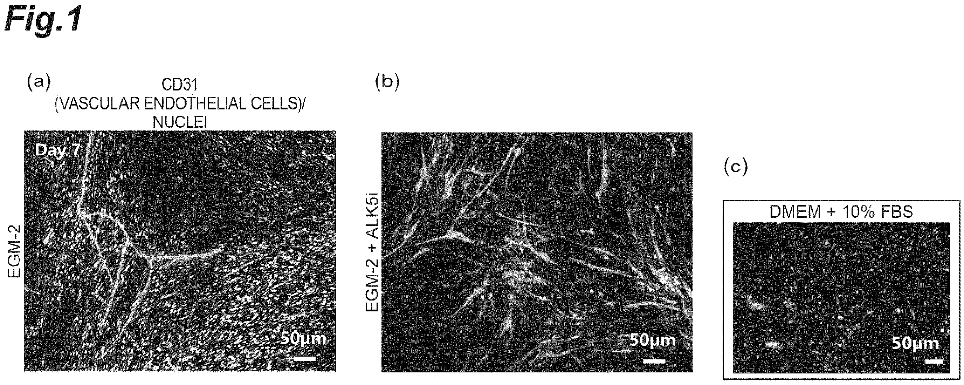

FIG. 1 shows the results obtained by carrying out fluorescence observations (20-fold magnification) of tissue constructs having a vascular network. (a) shows a tissue construct cultured in an EGM-2 medium that does not contain an ALK5 inhibitor (ALK5i), (b) shows a tissue construct cultured in an EGM-2 medium that contains a 5 µM ALK5 inhibitor, and (c) shows a tissue construct cultured in DMEM that contains 10% FBS. In (c), the formation of the vascular network could not be confirmed. It was confirmed that as compared with (a), the tissue construct in (b) has, like the biological tissue, blood vessels having a large diameter (for example, 10 µm or more and less than 25 µm) and blood vessels having a small diameter (for example, more than 0 µm and less than 10 µm), and a vascular network in which the number of branches is larger is formed.

It was shown that the culturing of adipose-derived stem cells in a culture medium containing ALK5 inhibitor promotes the differentiation of the adipose-derived stem cells into vascular endothelial cells.

### Production Example 2

Isolated ADSC was suspended in a culture medium of EGM-2 containing 0 µM or 5 µM of an ALK5 inhibitor (2-[3-(6-methyl-2-pyridinyl)-1H-pyrazol-4-yl]-1,5-naphthyridine, Cayman Chemical Company, No. 14794), seeded in a 10-cm dish, and incubated at 37°C.

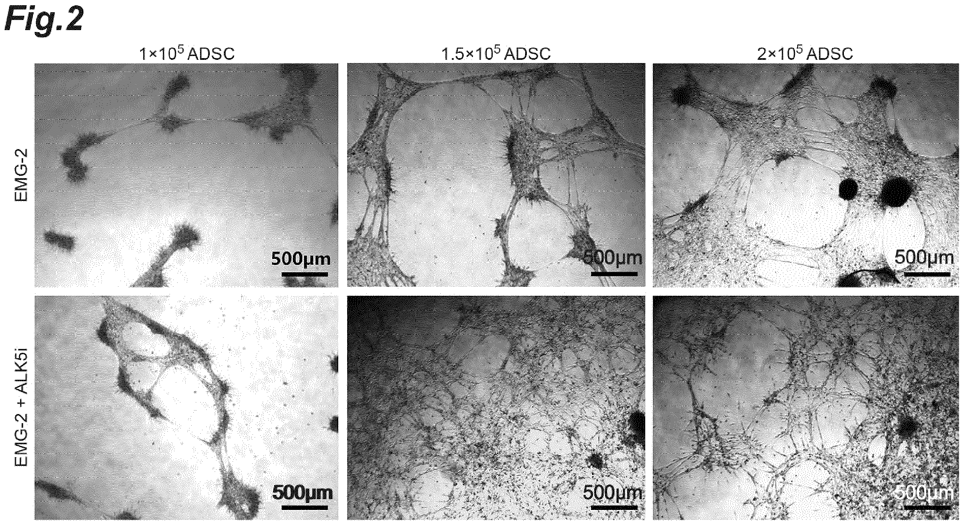

The culture medium was changed every 2 days, culture was carried out for 7 days. Thereafter, the ADSC was separated from the dish using trypsin/EDTA and suspended in 1 mL of DMEM, and the number of cells was counted. Further, the recovered ADSC was seeded on a 24-well plate previously coated with Matrigel at concentrations of 1.0 × 10⁵ cells/dish, 1.5 × 10⁵ cells/dish, and 2.0 × 10⁵ cells/dish, and incubated at 37°C for 24 hours. Thereafter, the morphology of the tissue construct was evaluated with a phase contrast microscope. FIG. 2 shows images of the produced tissue constructs, which are obtained by the phase contrast observation. It was observed that regardless of the number of ADSC cells, lumen formation is further progressed in the tissue construct cultured in EGM-2 that contains an ALK5 inhibitor (ALK5i) as compared with the tissue construct cultured in EGM-2 that does not contain an ALK5 inhibitor. In particular, it was suggested that due to the fact that it has been confirmed that a vascular network in which the number of branches of blood vessels is larger is formed in a case where the number of ADSC cells is 5 × 10⁵ cells/mL or more, the culturing of adipose-derived stem cells in a culture medium containing ALK5 inhibitor promotes the differentiation of the adipose-derived stem cells into vascular endothelial cells.

### Production Example 3

Isolated ADSC was suspended in a culture medium of EGM-2 containing 5 µM of an ALK5 inhibitor (2-[3-(6-methyl-2-pyridinyl)-1H-pyrazol-4-yl]-1,5-naphthyridine, Cayman Chemical Company, No. 14794), seeded in a 10-cm dish, and incubated at 37°C. The culture medium was changed every 2 days, and culture was carried out for 7 days to obtain a tissue construct. Thereafter, the ADSC was gently detached while leaving cell surface proteins, by incubation for 5 minutes using 0.25% Trypsin, recovered from the dish, and suspended in 1 mL of DMEM, and the number of cells was counted.

The ratio of vascular endothelial cells in the tissue construct was analyzed by the flow cytometry analysis using BD Facsmelody (registered trademark) cell sorter (Becton, Dickinson and Company). The tissue construct recovered as described above was centrifuged (100g, 5 minutes, 4°C). Thereafter, an anti-CD31 antibody labeled with Alexa Fluor 647 (100× dilution) was added thereto, and the cell suspension was further incubated at 4°C for 30 minutes. Thereafter, cells were centrifuged (100g, 5 minutes, 4°C) to wash the cells, and the pelleted cells were suspended in a buffer, and 10,000 events were recorded. Further, an isotype control for the conjugated antibody was measured simultaneously. Thereafter, analysis was carried out using Flowjo (registered trademark) Software (Becton, Dickinson and Company) to determine the ratio of CD31⁺ cells.

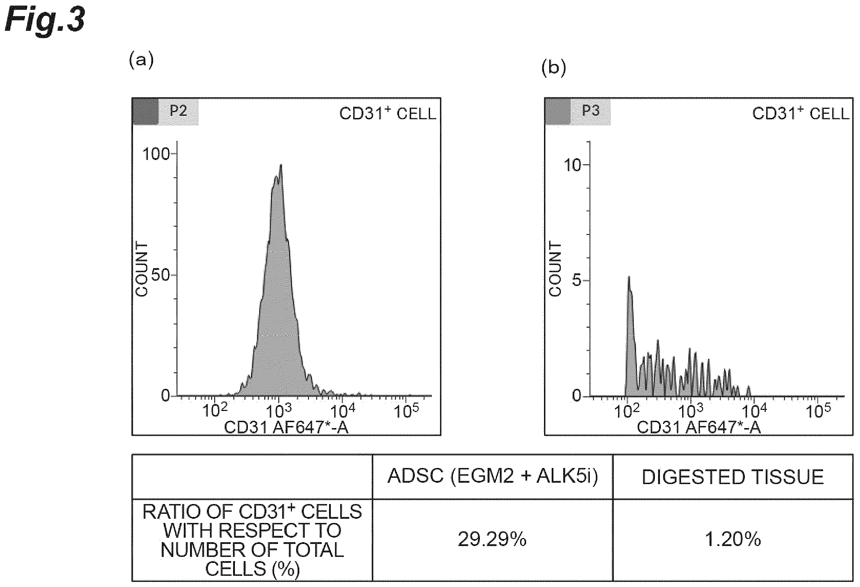

The results are shown in FIG. 3. (a) shows results obtained by using the produced tissue construct, and (b) shows results obtained by using cells as they are, the cells having been isolated after tissue digestion. In (a), it was found that 29.29% are vascular endothelial cells based on the number of total cells recovered from the tissue construct. On the other hand, in (b), only 1.20% were vascular endothelial cells.

### Production Example 4

A tissue construct (cell ball) produced by using the mature adipocytes, the ADSC, and the ADSC-derived vascular endothelial cells at a ratio of 2.5:1.5:1.5 (in terms of the number of cells) and a tissue construct (cell ball) produced by using the mature adipocytes and the ADSC-derived vascular endothelial cells at a ratio of 2.5:3 (in terms of the number of cells) were compared with a tissue construct (cell ball) produced by using the mature adipocytes, the ADSC, and the HUVEC at a ratio of 2.5:2:1 (in terms of the number of cells). In any of the tissue constructs, the total number of cells used is 4.0 × 10⁴ cells/ball. For the mature adipocytes and the ADSC, human-derived cells isolated as described above in the section of (Method for collecting mature adipocyte and ADSC) were used. For the ADSC-derived vascular endothelial cells, those obtained from the tissue construct prepared in Production Example 3 were used.

2.4 mg of the sCMF was weighed, 1 mL of DMEM was added thereto, and gentle mixing was carried out until only small particles of the sCMF were observed. Centrifugation was carried out at 10,000 rpm for 1 minute at room temperature, and the supernatant was aspirated to obtain a sCMF pellet. The ADSC and/or the ADSC-derived vascular endothelial cells (further, the HUVEC in the comparative example) were gently added onto the sCMF pellet, centrifugation was carried out at 3,500 rpm for 1 minute at room temperature, and the supernatant was aspirated. Fibrinogen (32 µL of a 50 mg/mL fibrinogen stock solution) was added and gently mixed with the cells and the sCMF. Further, mature adipocytes were added and gently mixed. A small amount of DMEM was added to adjust the total volume to 200 µL. Immediately, thrombin (3.9 mL of a 202 U/mL thrombin stock solution) was added and mixed a little, and then 5 µL (equivalent to one cell ball) of the mixture (equivalent to 40 cell balls) was seeded on each well of a low-adhesion 96-well plate (IWAKI #4860-800LP).

The gelation was carried out by incubation in a 37°C incubator for 15 minutes, and 300 µL of EGM-2 containing insulin at a final concentration of 10 µg/mL was added.

After 24 hours of culture, cells were transferred to a low-adhesion 24-well plate (IWAKI #4820-800LP), and 2 mL of the above EGM-2 was added thereto. The culture medium was changed every 2 to 3 days until the 7th day of culture.

Fluorescence imaging of the tissue construct was carried out according to the following procedure. A transwell containing the obtained tissue construct was transferred to a 24-well plate (IWAKI #4820-800LP). After washing with 200 µL of PBS, the tissue was fixed overnight under the condition of 4°C using 200 µL of 4% PFA. Washing was carried out three times using 200 µL of PBS. For immunostaining, cells were subjected to permeabilization treatment for 7 minutes under room temperature conditions by using 200 µL of 0.05% Triton/PBS, and washing was carried out three times using 200 µL of PBS.

The tissue was subjected to blocking treatment for 1 hour under room temperature conditions by using 200 µL of a 1% BSA/PBS solution. The BSA solution was aspirated, and 100 µL of a primary antibody solution (an anti-CD31 antibody diluted 100-fold in a 1% BSA/PBS solution) was added. A wet tissue paper was placed under the plate, and the plate was covered with an aluminum foil and incubated overnight under the condition of 4°C. After washing three times with 200 µL of PBS, a secondary antibody solution (an Alexa Fluor 647-labeled anti-mouse IgG antibody diluted 200-fold in a 1% BSA/PBS solution) was added thereto. A wet tissue paper was placed under the plate, the plate was covered with an aluminum foil and incubated for 2 hours under room temperature conditions, and then washing was carried out four times using 200 µL of PBS.

The cell ball was placed directly on a complete plate of a Confocal Quantitative Image Cytometer CQ1 (Yokogawa Electric Corporation), and the stained tissue construct was observed using the Confocal Quantitative Image Cytometer CQ1 with laser excitation light at 640 nm (Alexa Fluor 647).

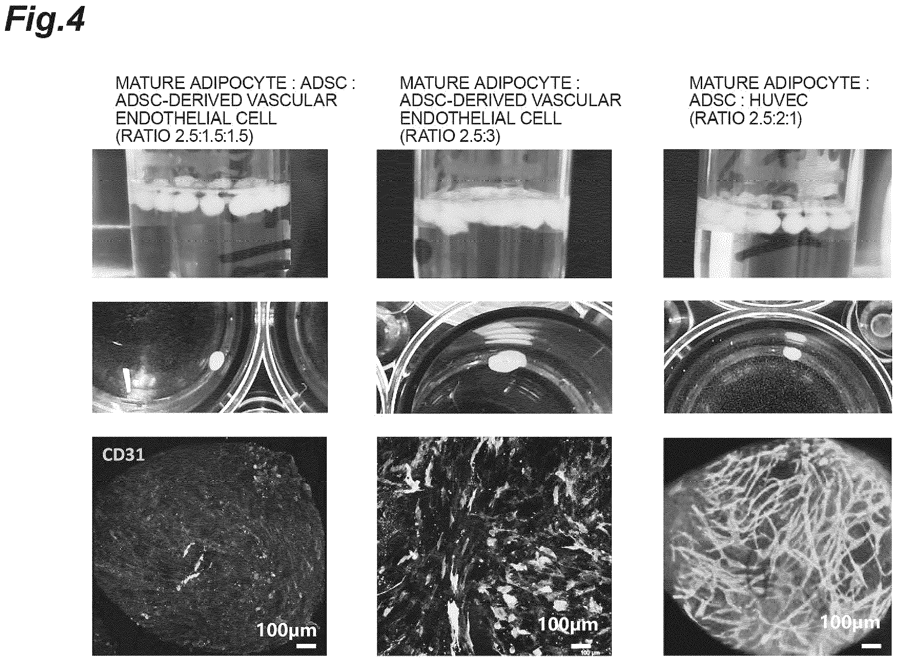

FIG. 4 shows the results obtained by observing cell balls having a vascular network by CD31 staining. In the cell balls obtained by using vascular endothelial cells obtained from the tissue construct in which the differentiation of adipose stem cells into vascular endothelial cells was promoted by an ALK5 inhibitor, the vascular network was formed as in the case of the tissue construct prepared using the HUVEC regardless of whether or not the ADSC was used and regardless of the ratio of various cell types. These vascular networks were formed up to the inside of the cell balls.

### Production Example 5

Isolated ADSC was suspended in a culture medium of EGM-2 containing 0 µM or 5 µM of an ALK5 inhibitor (2-[3-(6-methyl-2-pyridinyl)-1H-pyrazol-4-yl]-1,5-naphthyridine, Cayman Chemical Company, No. 14794), seeded in a 10-cm dish, and incubated at 37°C. The culture medium was changed every 2 days, and culture was carried out for 7 days to obtain a tissue construct. Thereafter, the ADSC was gently detached while leaving cell surface proteins, by incubation for 5 minutes using 0.25% Trypsin, recovered from the dish, and suspended in 1 mL of DMEM, and the number of cells was counted.

The ratio of vascular endothelial cells in the sample was analyzed by the flow cytometry analysis using BD Facsmelody (registered trademark) cell sorter (Becton, Dickinson and Company). The sample recovered as described above was centrifuged (100g, 5 minutes, 4°C). Thereafter, an anti-CD31 antibody labeled with Alexa Fluor 647 (100× dilution) was added thereto, and the cell suspension was further incubated at 4°C for 30 minutes. Thereafter, cells were centrifuged (100g, 5 minutes, 4°C) to wash the cells, and the pelleted cells were suspended in a buffer, and 10,000 events were recorded. Further, an isotype control for the conjugated antibody was measured simultaneously. Thereafter, analysis was carried out using Flowjo (registered trademark) Software (Becton, Dickinson and Company) to determine the ratio of CD31⁺ cells.

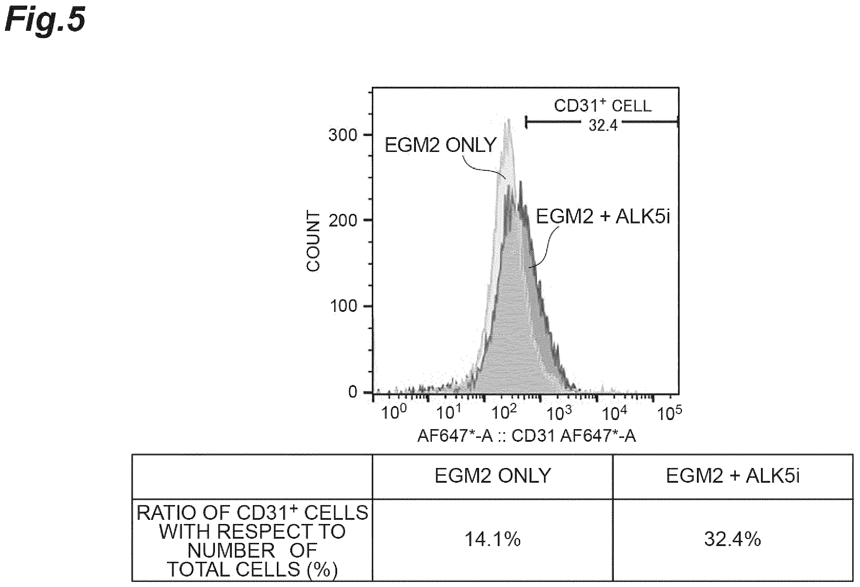

The results are shown in FIG. 5. In the sample cultured in an EGM2 medium containing 5 µM of an ALK5 inhibitor, it was found that 32.4% are vascular endothelial cells based on the number of total cells recovered. On the other hand, in the sample cultured in an EGM2 medium that does not contain an ALK5 inhibitor, it was shown that 14.1% are vascular endothelial cells, and the ALK5 inhibitor remarkably has induced the differentiation of the ADSC into vascular endothelial cells.

Isolated ADSC was suspended in an EGM-2 medium, seeded in a 10-cm dish, and incubated at 37°C. The culture medium was changed every 2 days, and culture was carried out for 7 days to obtain a tissue construct. From this tissue construct, cells including ADSC, ADSC-derived vascular endothelial cells, and vascular endothelial progenitor cells were recovered.

The above-described recovered cells, the sCMF, fibrin, the mature adipocytes, and thrombin were mixed by the same method as in Production Example 4, and 5 µL (equivalent to one cell ball) of the mixture (equivalent to 40 cell balls) was seeded on each well of a low-adhesion 96-well plate (IWAKI #4860-800LP). The total number of cells of the tissue construct (cell ball) used was 4.0 × 10⁴ cells/ball, and the mature adipocytes and the above-described recovered cells were used a ratio of 3:2.5 for the production.

The cell ball seeded in the low-adhesion 96-well plate was incubated in a 37°C incubator for 15 minutes to carry out gelation, and 300 µL of EGM-2 or KBM was added thereto. It is noted that For the above EGM-2 or KBM, EGM-2 or KBM, which had been adjusted to contain insulin of a final concentration of 10 µg/mL, a 5 µM ALK-5 inhibitor (2-[3-(6-methyl-2-pyridinyl)-1H-pyrazol-4-yl]-1,5-naphthyridine, Cayman Chemical Company, No. 14794), and VEGF of 0.5 ng/mL, 20 ng/mL, 50 ng/mL, or 100 ng/mL, or leptin of 1 µg/mL, was used. In addition, EGM-2 or KBM, containing only insulin of a final concentration of 10 µg/mL and an ALK-5 inhibitor of 5 µM, was used as a control.

After 24 hours of culture, cells were transferred to a low-adhesion 24-well plate (IWAKI #4820-800LP), and 2 mL of the above EGM-2 or KBM was added thereto. The culture medium was changed every 2 to 3 days until the 7th day of culture.

The fluorescence imaging of the tissue construct was carried out as in Production Example 4, except that an anti-von Willebrand factor (vWF) antibody diluted 100-fold in a 1% BSA/PBS solution was used as a primary antibody solution and an Alexa Fluor 647-labeled anti-rabbit IgG antibody solution diluted 200-fold in 1% BSA/PBS solution was used as a secondary antibody solution. The vWF was used as a marker for vascular endothelial cells. In addition, for observation, the cell ball was placed directly on a complete plate of a Confocal Quantitative Image Cytometer CQ1 (Yokogawa Electric Corporation), and the stained tissue construct was observed using the Confocal Quantitative Image Cytometer CQ1 with laser excitation light at 640 nm.

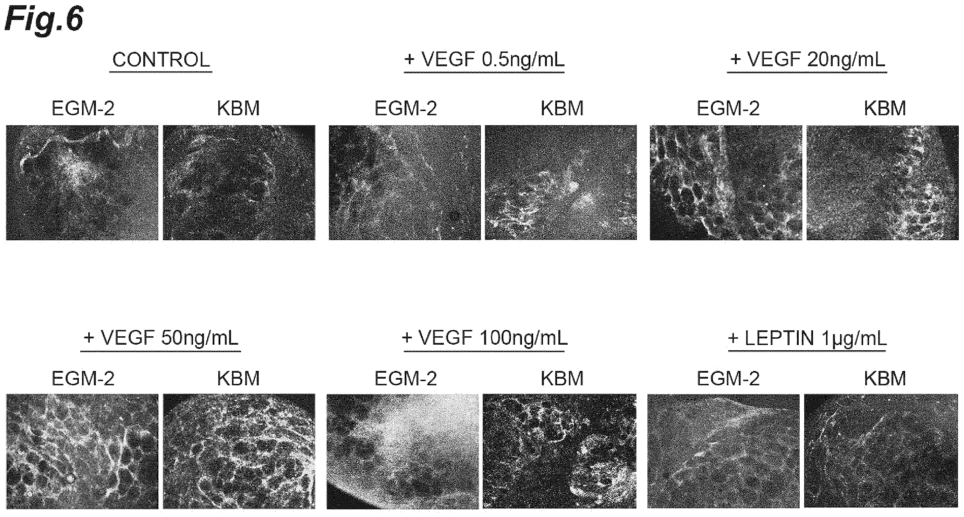

FIG. 6 shows the results obtained by observing cell balls having a vascular network by vWF staining. In the cell balls cultured in the culture medium containing VEGF a vascular network, in which the number of branches of blood vessels is large as compared with the control cell ball cultured in a culture medium which does not contain VEGF, was formed. In addition, in the cell balls cultured in the culture medium containing VEGF, a vascular network, in which the number of branches of blood vessels is large as compared with the cell ball cultured in a culture medium containing leptin, was formed. Among the cell balls cultured in the culture medium to which VEGF was added, by carrying out culture in a culture medium, the culture medium containing more than 20 ng/mL of VEGF.a vascular network having still more blood vessels was formed

From these results, it was shown that in a case where VEGF is added to a culture medium in addition to the ALK-5 inhibitor, the differentiation of adipose-derived stem cells from human into vascular endothelial cells and angiogenesis are further promoted.

### Production Example 7

A tissue construct (cell ball) was produced by the same method as in Production Example 6. Regarding the produced tissue construct, 5 µL (equivalent to one cell ball) of the mixture (equivalent to 40 cell balls) was seeded on each well of a low-adhesion 96-well plate (IWAKI #4860-800LP).

The cell ball seeded in the low-adhesion 96-well plate was incubated in a 37°C incubator for 15 minutes to carry out gelation, and 300 µL of EGM-2 containing insulin of a final concentration of 10 µg/mL, an AKL-5 inhibitor of 5 µM, and VEGF of 50 ng/mL was added thereto.

After 24 hours of culture, cells were transferred to a low-adhesion 24-well plate (IWAKI #4820-800LP), and 2 mL of the above EGM-2 or KBM was added thereto. The culture medium was changed every 2 to 3 days until the 7th day of culture.

The fluorescence imaging of the tissue construct was carried out as in Production Example 4, except that various primary antibody solutions and the corresponding secondary antibody solutions were used. For the primary antibody solution, the following antibody was used: a rabbit anti-CD34 antibody diluted 100-fold in a 1% BSA/PBS solution, a mouse anti-CD31 diluted 100-fold in a 1% BSA/PBS solution, or a rabbit anti-von Willebrand factor (vWF) antibody diluted 100-fold in a 1% BSA/PBS solution. For the secondary antibody solutions, the following antibodies were used: an Alexa Fluor 546-labeled anti-mouse IgG antibody diluted 200-fold in a 1% BSA/PBS solution, and an Alexa Fluor 647-labeled anti-rabbit IgG antibody diluted 200-fold in a 1% BSA/PBS solution. CD34 was used as a marker for vascular endothelial progenitor cells. CD31 was used as a marker for vascular endothelial progenitor cells and vascular endothelial cells. The vWF was used as a marker for vascular endothelial cells. In addition, nuclei were also stained by Hoechst staining.

For observation, the cell ball was placed directly on a complete plate of a Confocal Quantitative Image Cytometer CQ1 (Yokogawa Electric Corporation), and the stained tissue construct was observed using the Confocal Quantitative Image Cytometer CQ1. The laser excitation light for Alexa Fluor 546 was set at 556 nm, and the laser excitation light for Alexa Fluor 647 was set at 640 nm.

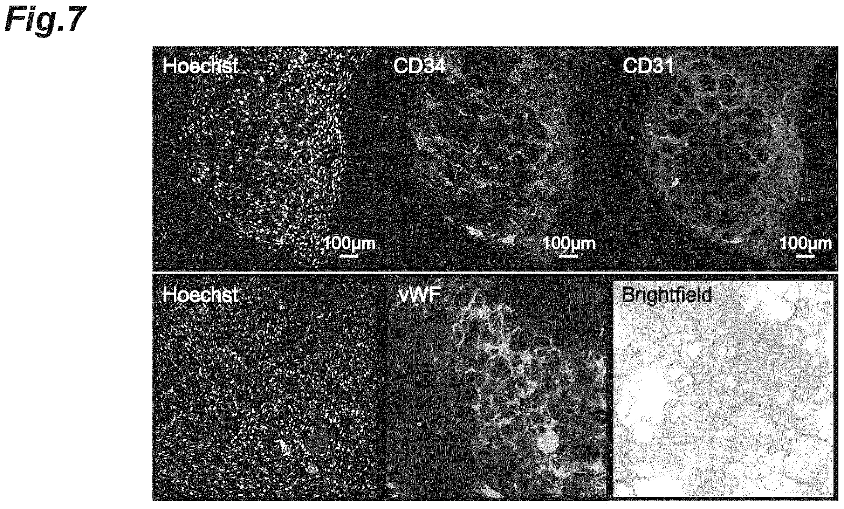

The results are shown in FIG. 7. Due to the addition of VEGF to the culture medium, the proportion of vWF-positive cells with respect to the total CD31-positive cells was higher than the proportion of CD34-positive cells. In other words, the number of vascular endothelial cells was larger than the number of vascular endothelial progenitor cells in the cell ball produced as described above. In addition, a vascular network was also formed.

From these results, it was shown that in a case where VEGF is added to a culture medium in addition to the ALK-5 inhibitor, the differentiation of adipose-derived stem cells from human into vascular endothelial cells and angiogenesis are further promoted.

## Claims

1. A method for producing a tissue construct comprising vascular endothelial cells, the method comprising incubating cells comprising at least adipose-derived stem cells from human in the presence of a TGFβ type I receptor inhibitor.

2. The method according to Claim 1, wherein the cells comprising at least adipose-derived stem cells from human comprise no vascular endothelial cells.

3. The method according to Claim 1, wherein the incubating in the presence of the TGFβ type I receptor inhibitor is incubating in a culture medium comprising the TGFB type I receptor inhibitor, and,
a content of the TGFβ type I receptor inhibitor in the culture medium is 1 µM or more and 10 µM or less.

4. The method according to Claim 1, wherein the incubating is performed for 96 hours or more and 288 hours or less.

5. The method according to Claim 1, comprising incubating the cells together with a fragmented extracellular matrix component in the presence of the TGFβ type I receptor inhibitor.

6. The method according to Claim 5, wherein in the tissue construct, the fragmented extracellular matrix component is disposed in gaps between the cells.

7. The method according to Claim 5, wherein the fragmented extracellular matrix component is a fragmented collagen component.

8. The method according to any one of Claims 5 to 7, further comprising, before the incubation, a step of bringing the cells into contact with the fragmented extracellular matrix component in an aqueous medium.

9. A method for promoting differentiation of adipose-derived stem cells into vascular endothelial cells, the method comprising incubating cells comprising at least adipose-derived stem cells from human in the presence of a TGFβ type I receptor inhibitor.

10. The method according to Claim 9, wherein the incubating in the presence of the TGFβ type I receptor inhibitor is incubating in a culture medium comprising the TGFB type I receptor inhibitor, and,
a content of the TGFβ type I receptor inhibitor in the culture medium is 1 µM or more and 10 µM or less.

11. The method according to Claim 9, wherein the incubating is performed for 96 hours or more and 288 hours or less.

12. The method according to Claim 9, comprising incubating the cells comprising at least the adipose-derived stem cells, together with a fragmented extracellular matrix component in the presence of the TGFβ type I receptor inhibitor.

13. The method according to Claim 12, wherein the fragmented extracellular matrix component is a fragmented collagen component.

14. The method according to Claim 12 or 13, further comprising, before the incubation, a step of bringing the cells into contact with the fragmented extracellular matrix component in an aqueous medium.
